# EUROPÄISCHE PATENTSCHRIFT

(11) **EP 0 699 081 B1**
(45) Veröffentlichungstag und Bekanntmachung des Hinweises auf die Patenterteilung: **17.03.1999**
(21) Anmeldenummer: 94916975.9
(22) Anmeldetag: 19.05.1994
(51) Int. Cl.: A61L 29/00, A61M 16/04

(54) **ENDOTRACHEALER TUBUS**
ENDOTRACHEAL TUBE
TUBE ENDOTRACHEEN

(30) Priorität: 20.05.1993 DE 4316920
(43) Veröffentlichungstag der Anmeldung: 06.03.1996
(73) Patentinhaber: HARTMANN, Michael, D-79232 March (DE); MÜLLER, Bert, CH-1020 Renens (CH)
(72) Erfinder: HARTMANN, Michael, D-79232 March (DE); MÜLLER, Bert, CH-1020 Renens (CH)
(74) Vertreter: König, Norbert, Dipl.-Phys. Dr.
(86) Internationale Anmeldenummer: EP9401621
(87) Internationale Veröffentlichungsnummer: WO9427652

(56) Entgegenhaltungen:
- EP-A- 0 206 024
- EP-A- 0 301 717
- EP-A- 0 302 186
- EP-A- 0 318 258
- AU-B- 558 588
- FR-A- 2 109 932
- US-A- 4 612 337

## Beschreibung

Die Erfindung betrifft einen endotrachealen Tubus gemäß Oberbegriff des Anspruchs 1.

Bei der künstlichen Beatmung besteht die Gefahr einer Lungenentzündung durch entlang dem Tubus in die Lunge gelangende Keime.

Durch die AU 558 588 ist ein endotrachealer Tubus bekannt, der mit einem antimikrobiell wirkenden Mittel versehen ist, das auf die Innenfläche des Tubus aufgebracht und durch ein oder mehrere organische Substanzen gebildet ist. Nachteilig bei diesem bekannten Tubus ist, daß das antimikrobiell wirkende Mittel lediglich gegen durch das Innere des Tubus, beispielsweise mit der Beatmungsluft, eingeschleppte Keime wirksam ist. Ein Schutz gegen entlang der Außenfläche des Tubus eingeschleppte Keime besteht nicht. Ein weiterer Nachteil des bekannten Tubus besteht darin, daß die organischen Substanzen, die das antimikrobell wirkende Mittel bilden, insbesondere in höheren Konzentrationen, zu einer gesundheitlichen Beeinträchtigung des Patienten führen können.

Durch die EP-PS 206 024 sind medizinische Geräte, beispielsweise Katheter bekannt, die mit einem antimikrobiell wirkenden Mittel versehen sind, das durch wenigstens zwei Metallschichten gebildet ist, die miteinander in elektrisch leitender Verbindung stehen und von denen wenigstens eine mit der Außenfläche des Katheters verbunden ist. Eine der Schichten wird durch ein Edelmetall gebildet, während die andere Schicht durch ein Nichtedelmetall gebildet wird. Nachteilig ist, daß die Herstellung des Katheters aufgrund der wenigstens zwei erforderlichen Schichten aufwendig und teuer ist, zumal die beiden Schichten nahezu die gesamte Außenfläche des Katheters bedecken sollen.

Die Aufgabe der vorliegenden Erfindung besteht deshalb darin, einen endotrachealen Tubus so auszubilden, daß die Gefahr einer Lungenentzündung durch entlang dem Tubus eingeschleppte Keime weitgehend vermieden ist.

Diese Aufgabe wird durch die Ausbildung gemäß Kennzeichen des Anspruchs 1 gelöst.

Vorteilhafte und zweckmäßige Weiterbildungen der erfindungsgemäßen Aufgabenlösung sind in den Unteransprüchen angegeben.

Durch die erfindungsgemäße Ausbildung wird der endotracheale Tubus mit einer Barriere für Keime vorgesehen. Für die Ausbildung dieser Barriere liegt der Erfindung die bekannte Erkenntnis zugrunde, daß bestimmte Substanzen, wie Silber und Silbersalze, bereits in Spuren stark bakterizide und bakteriostatische Wirkung haben. Bei Silber und Silbersalzen sind es die Silber-Ionen, die diese Wirkung haben. Durch das erfindungsgemäß vorgeschlagene Aufbringen von antimikrobiell wirkenden Mitteln, beispielsweise von Silber oder Silbersalzen, auf eine oder mehrere Umfangsstellen des Tubus oder durch Einsetzen eines entsprechend ausgebildeten Rohrstückes werden automatisch antimikrobielle Substanzen, beispielsweise antimikrobielle Silber-Ionen, erzeugt, und somit wird automatisch eine antimikrobielle Sphäre um den Tubus herum geschaffen, die das Eindringen von Keimen entlang dem Tubus in die Lunge verhindert.

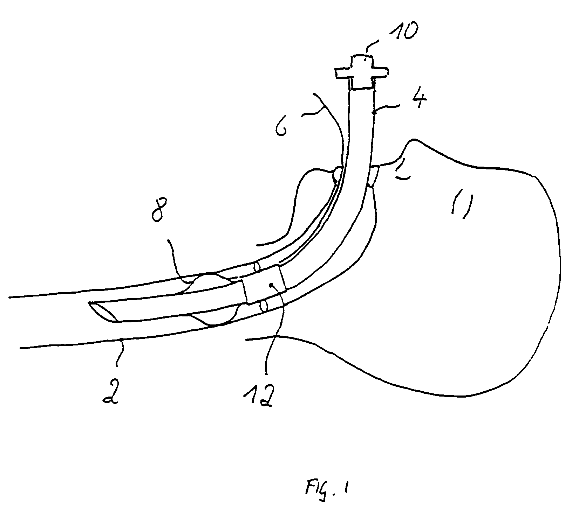

Die Erfindung soll nachfolgend anhand der beigefügten Zeichnung, die ein Ausführungsbeispiel zeigt, näher erläutert werden.

Die Zeichnung zeigt schematisch einen in die Luftröhre 2 des Menschen eingeführten endotrachealen Tubus 4, der mit einer über einen Schlauch 6 aufblasbaren Blockermanschette 8 versehen ist und an seinem äußeren Ende ein Verbindungsstück 10 für einen nicht dargestellten Beatmungsschlauch aufweist.

Auf dem Umfang des Tubus 4 ist an einer vor der Blockermanschette 8 befindlichen Stelle ein antimikrobiell wirkendes Mittel, beispielsweise in Form einer Silberfolie, einer Silberbeschichtung oder einer Silbersalzbeschichtung angeordnet. Die Silberbeschichtung kann durch Aufdampfen hergestellt sein. Es können an mehreren Stellen des Tubus solche antimikrobiell wirkenden Mittel vorgesehen werden.

Beim dargestellten Beispiel befindet sich die antimikrobielle Stelle unmittelbar vor der Blockermanschette 8 im Bereich der Stimmritzen 14.

Es ist auch möglich, die Außenfläche der Blockermanschette 8 mit dem Mittel 12, also mit der Silberfolie, der Silberbeschichtung oder der Silbersalzbeschichtung zu versehen.

Das antimikrobiell wirkende Mittel 12 kann auch ein Rohrstück sein, das in den Tubus eingesetzt ist und das aus Silber beteht, versilbert ist oder als Träger einer antimikrobiellen Substanz, beispielsweise eines Silbersalzes, ausgebildet ist.

Durch das Silber oder das Silbersalz wird ein endotrachealer Tubus mit antimikrobiellen Eigenschaften geschaffen. Da sich zwischen Tubus und Luftröhre stets Speichel ansammelt, das Silber oder das Silbersalz also ständig von einem flüssigen Medium umgeben ist, gehen Silber-Ionen, welche die bekannte antimikrobielle Wirkung haben, automatisch in Lösung.

Es ist auch möglich, Silber-Ionen durch Anlegen einer Gleichspannung aus der Silberfolie oder der aufgedampften Silberbeschichtung oder dem Rohrstück herauszulösen, indem das Silber oder das Rohrstück als Lösungselektrode (Anode) geschaltet wird.

## Patentansprüche

1. Endotrachealer Tubus, welcher wenigstens eine Manschette aufweist und mit einem antimikrobiell wirkenden, Metallionen im umgebenden Medium freisetzenden Mittel versehen ist, **dadurch gekennzeichnet**, daß das Mittel (12) eine an einer oder mehreren Umfangsstellen auf dem Tubus (4) aufgebrachte Silberbedampfung oder Silbersalzbeschichtung oder eine um den Tubus herum angeordnete oder als Rohrstück in den Tubus eingesetzte, einen Teil des Tubus bildende Folie ist, die aus Silber besteht oder silberbedampft ist oder mit einer Silbersalzaußenbeschichtung versehen ist.

2. Endotrachealer Tubus nach Anspruch 1, **dadurch gekennzeichnet**, daß an das als Anode geschaltete Mittel (12) eine Gleichspannung angelegt ist.

3. Endotrachealer Tubus nach Anspruch 1 oder 2, **dadurch gekennzeichnet**, daß das antimikrobiell wirkende Mittel (12) vor der Manschette (8) angeordnet ist.

4. Endotrachealer Tubus nach Anspruch 1,2 oder 3, **dadurch** **gekennzeichnet**, daß das antimikrobiell wirkende Mittel auf der Manschette (8) angeordnet ist.

5. Endotrachealer Tubus nach einem der vorhergehenden Ansprüche, **dadurch gekennzeichnet**, daß mehrere der Mittel kombiniert an mehreren Stellen des Tubus (4) angeordnet sind.

## Claims

1. An endotracheal tube which has at least a collar and is provided with an anti-microbe acting means which releases metal ions into the surrounding medium, characterised in that the means (12) is a silver vapour deposit or silver salt coating applied at one or more circumferential places on the tube (4), or a sheet arranged around the tube or inserted as a pipe piece of the tube, which is comprised of silver or is silver vapour deposited, or is provided with a silver salt outer coating.

2. An endotracheal tube according to claim 1, characterised in that a direct voltage is applied to the means (12) connected as an anode.

3. An endotracheal tube according to claims 1 or 2, characterised in that the anti-microbe acting means (12) is arranged in front of collar (8).

4. An endotracheal tube according to claims 1,2 or 3, characterised in that the anti-microbe acting means (12) is arranged on the collar (8).

5. An endotracheal tube according to any of the above claims, characterised in that a plurality of means are arranged combined at a plurality of positions on the tube.

## Revendications

1. Tube endotrachéen qui présente au moins une manchette et est muni d'un agent à action antimicrobienne dégageant des ions métalliques dans le milieu ambiant,
caractérisé en ce que l'agent (12) est une vaporisation sous vide d'argent ou un revêtement de sel d'argent, déposé en un ou plusieurs emplacements périphériques sur le tube (4), ou bien une feuille disposée autour du tube ou logée dans le tube sous forme de morceau de tube, formant une partie du tube, qui est constituée d'argent ou est formée d'un dépôt sous vide d'argent ou est munie d'un revêtement extérieur en sel d'argent.

2. Tube endotrachéen selon la revendication 1,
caractérisé en ce qu'une tension continue est appliquée à l'agent (12) monté en tant qu'anode.

3. Tube endotrachéen selon la revendication 1 ou 2,
caractérisé en ce que l'agent (12) à action antimicrobienne est disposé avant la manchette (8).

4. Tube endotrachéen selon la revendication 1, 2 ou 3,
caractérisé en ce que l'agent à action antimicrobienne est disposé sur la manchette (8).

5. Tube endotrachéen selon l'une des revendications précédentes,
caractérisé en ce que plusieurs agents sont disposés combinés à plusieurs emplacements du tube (4).
